# EUROPEAN PATENT APPLICATION

(11) **EP 2 710 901 A1**
(43) Date of publication of application: **26.03.2014**
(21) Application number: 12185153.9
(22) Date of filing: 20.09.2012
(51) Int. Cl.: A23L 1/30, A61K 35/74, A61K 36/00

(54) **Dietary supplement compositions**

(71) Applicant: Symrise AG, 37603 Holzminden (DE)
(72) Inventor: Krammer, Gerhard, 37603 Holzminden (DE); Widder, Sabine, 37603 Holzminden (DE)
(74) Representative: Fabry, Bernd

(57) **Abstract**

Suggested is a dietary supplement composition, comprising
(a) at least one probiotic micro-organism and
(b) at least one anthocyanin or a plant extract or a fruit juice comprising anthocyanins, and optionally
(c) at least one prebiotic
on condition that the compositions are free of milk fermentation products.

## Description

### Field of invention

The present invention relates to the area of food additives and is directed to dietary supplements comprising probiotics and a defined group of flavonoids for improving the health status of the body and in particular the health of the intestine.

### State of the art

Probiotics contain live bacteria and represent an important part of the complex world of foods that are good for health. It is the bacteria and the metabolites which they produce that give these products their health promoting properties. The best known example of a probiotic is yoghurt. The experimental data for yoghurt is still not as conclusive as one would like, however, human studies related to the consumption of dietary milk products show increased milk digestibility, quicker recovery from certain types of diarrhoea, enhanced immune function, relation in certain cancers, and possible lowering of blood cholesterol levels.

In this context reference is made to Italian patent IT 1373036 B1 referring to a composition for oral administration, particularly a food supplement for the treatment and prevention of infection by Helicobacter pylori in humans, comprising a milk fermentation product, fruits selected from blueberry, currant, black currant, blackberry, raspberry, strawberry, grape, pomegranate, and cherry, and optionally probiotics.

EP 1281403 A1 (Lab Dolisos) discloses pharmaceutical and/or dietetic compositions containing 0.1 - 30 % b.w. of a vegetable extract, and living probiotic micro-organisms, that are given simultaneously, separately or sequentially to man or animals, with the proviso that the vegetable is not soya.

From EP 2168441 A1 (Doehler) probiotic smoothies are known comprising at least 95 % b.w. of fruit- and/or vegetable preparations, 0.005-5 % b.w. of a mixture of at least two ingredients comprising antioxidants, fermentation products and food extracts, and at least 10⁶ CFU/ml of probiotic germs, where the product has a pH-value of greater than 4-4.6.

Bacteria found in products like yoghurt, kefir or fermented vegetables often aren't found in the human intestine. In fact, the intestinal environment is often a hostile one for these foreign bacteria. Because of this, bacteria eaten in probiotic products don't colonise the intestine but are flushed through and eliminated from the body.

The bacteria living in the intestine make up a very large and very diverse population. The numbers of each kind of bacteria change depending on age, diet, health status, and use of drugs and supplements. The effects are linked to the ability of the bacteria to adhere to the intestinal wall and use the semi-digested food that it passing through the intestines. There are a lot of different health benefits linked to the administration of probiotics to the human body which are reported in the literature, such as
- reduction *of Heliobacter pylori*,
- reduction of allergic symptoms,
- relief from constipation,
- relief from inflammatory bowel syndrome,
- beneficial effects on mineral metabolism, particularly bone density and stability (osteoporosis prevention),
- reduction of cholesterol and triacylglycerol plasma concentrations, and especially
- prevention of intestine cancer.

However, probiotics also show some disadvantages. For example, in case of inflammatory diseases of the intestine the mucosa epidermis becomes permeable for probiotic bacteria, so that they can enter into the serum. The same is true for elderly people. Also in case of a medicamentation with antibiotics the administration of probiotics is not suggested in order to allow the intestine flora de regenerate without being mixed up with probiotic bacteria which cannot settle in the intestine.

Therefore, the object of the present invention has been to provide a suitable additive for the administration of probiotics to the human body providing the same health benefits without being linked with the disadvantages as described above.

### Description of the invention

Object of the present invention are dietary supplement compositions comprising
(a) at least one probiotic micro-organism and
(b) at least one anthocyanin or a plant extract or a fruit juice comprising anthocyanins, and optionally
(c) at least one prebiotic
on condition that the compositions are free of milk fermentation products.

Surprisingly it has been observed that probiotics and anthocyanes showing synergistic behaviour in terms of health benefits. In Particular, adding anthocyanins, which show a strong anti-oxidative effect, improve the performance of probiotic bacteriae. In particular it was found that by adding anthocyanins the concentration of toxic metabolism products was significantly reduced.

### Probiotics

Probiotic organisms, also called "probiotics" forming component (a) represent live micro-organisms which are considered to be beneficial to the host organism. According to the currently adopted definition by FAO/WHO, probiotics are: "Live microorganisms which when administered in adequate amounts confer a health benefit on the host". Lactic acid bacteria (LAB) and bifidobacteria are the most common types of microbes used as probiotics; but certain yeasts and bacilli may also be used. Probiotics are commonly consumed as part of fermented foods with specially added active live cultures; such as in yogurt, soy yogurt, or as dietary supplements. Live probiotic cultures are available in fermented dairy products and probiotic fortified foods. However, tablets, capsules, powders and sachets containing the bacteria in freeze dried form are also available. Table 1 provides an overview of common probiotics and their respective health claims that can be used as component (a) of the present invention:

**Table 1**

| Probiotics | | | |
|---|---|---|---|
| **Strain** | **Brand name** | **Producer** | **Health claims** |
| *Bacillus coagulans* GBI-30,6086 | GanedenBC | Ganeden Biotech | May improve abdominal pain and bloating in IBS patients. May increase immune response to a viral challenge. |
| *Bifidobacterium animalis* subsp. *lactis* BB -12 | Probio-Tec Bifidobacterium BB-12 | Chr. Hansen | Human studies have shown that BB-12 alone or in combination may have an effect on the gastrointestinal system. |
| *Bifidobacterium infantis* 35624 | Align | Procter & Gamble | In one preliminary study, showed possible improvement for abdominal pain/discomfort and bowel movement difficulty. |
| *Lactobacillus acidophilus* NCFM | | Danisco | Shown in one study to reduce the side effects of antibiotic therapy. |
| *Lactobacillus paracasei* St11 (or NCC2461) | | | |
| *Lactobacillus johnsonii* La1 (= Lactobacillus LC1, *Lactobacillus johnsonii* NCC533) | | Nestlé | May reduce incidence of H pylori-caused gastritis and may reduce inflammation |
| *Lactobacillus plantarum* 299v | GoodBelly/ Pro-Viva/ProbiMage | Probi | May improve symptoms of IBS; however, more research is required. |
| *Lactobacillus reuteri* American Type Culture Collection\| ATTC 55730 (*Lactobacillus reuteri* SD2112) | | BioGaia | Preliminary evidence for diarrhea mitigation in children, H. pylori infection, possible effect on gingivitis, fever in children and number of sick days in adults. |
| *Lactobacillus reuteri* Protectis (DSM 17938, daughter strain of ATCC 55730) | | | |
| *Saccharomyces boulardii* | DiarSafe and others | Wren Laboratories | Limited evidence for treatment of acute diarrhea. |
| *Lactobacillus rhamnosus* GR-1 & *Lactobacillus reuteri* RC-14 | Bion Flore Intime/ Jarrow Fem-Dophilus | Chr. Hansen | In one study, oral ingestion resulted in vaginal colonisation and reduced vaginitis. |
| *Lactobacillus acidophilus* NCFM *& Bifidobacterium bifidum* BB-12 | Florajen3 | American Lifeline, Inc | Preliminary evidence for reduced C. difficile-associated disease (CDAD). |
| *Lactobacillus acidophilus* CL1285 *& Lactobacillus casei LBC80R* | Bio-K+ CL1285 | Bio-K+ International | May affect digestive health. In vitro inhibition of *Listeria monocytogenes* and *L. innocua, Escherichia coli, Staphylococcus aureus, Enterococcus faecalis* and *Enterococcus faecium.* Reduction of symptoms of lactose intolerance and immune stimulation_{.}^{[86]} |
| *Lactobacillus plantarum* HEAL 9 *& Lactobacillus paracasei* 8700:2 | Bravo Friscus/ ProbiFrisk | Probi | Is under study for common cold infections. |

Some additional forms of lactic acid bacteria, representing also suitable probiotics include:
- *Lactobacillus bulgaricus;*
- *Streptococcus thermophilus;*
- *"Lactobacillus bifidus" -* became new genus *Bifidobacterium.*

Some fermented products containing similar lactic acid bacteria include:
- Pickled vegetables
- Fermented bean paste such as tempeh, miso and doenjang;
- Kefir;
- Buttermilk or Karnemelk;
- Kimchi;
- Pao cai;
- Soy sauce;
- Zha cai.

### Anthocyanins

Anthocyanins, also called anthocyans, forming component (b) represent water-soluble vacuolar pigments that may appear red, purple, or blue according to the pH. They belong to the class of flavonoids synthesized via the phenylpropanoid pathway following general formula (I)

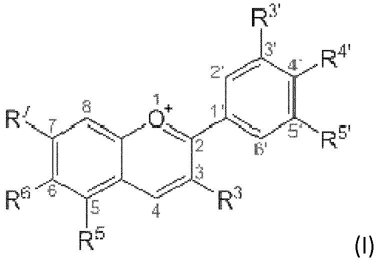

Anthocyanins are odourless and nearly flavourless, contributing to taste as a moderately astringent sensation. Anthocyanins occur in all tissuesof higher plants, including leaves, stems, roots, flowers, and fruits. Anthoxanthins are their clear, white to yellow counterparts occurring in plants. Anthocyanins are derivatives of anthocyanidins, which include pendant sugars. Most frequent in nature are the glycosides of cyanidin, delphinidin, malvidin, pelargonidin, peonidin, and petunidin. Roughly 2% of all hydrocarbons fixated in photosynthesis are converted into flavonoids and their derivatives such as the anthocyanins. Table 2 provides an overview of the most common anthocyanin species.

**Table 2**

| Anthocyanins | | | | | | | | |
|---|---|---|---|---|---|---|---|---|
| **Anthocyanin** | **Basic structure** | **R_{3'}** | **R_{4'}** | **R_{5'}** | **R₃** | **R₅** | **R₆** | **R₇** |
| Aurantinidin | 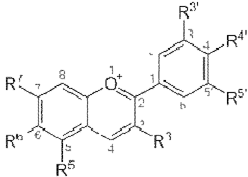 | -H | -OH | -H | -OH | -OH | -OH | -OH |
| Cyanidin | | -OH | -OH | -H | -OH | -OH | -H | -OH |
| Delphinidin | | -OH | -OH | -OH | -OH | -OH | -H | -OH |
| Europinidin | | -OCH₃ | -OH | -OH | -OH | -OCH₃ | -H | -OH |
| Luteolinidin | | -OH | -OH | -H | -H | -OH | -H | -OH |
| Pelargonidin | | -H | -OH | -H | -OH | -OH | -H | -OH |
| Malvidin | | -OCH₃ | -OH | -OCH₃ | -OH | -OH | -H | -OH |
| Peonidin | | -OCH₃ | -OH | -H | -OH | -OH | -H | -OH |
| Petunidin | | -OH | -OH | -OCH₃ | -OH | -OH | -H | -OH |
| Rosinidin | | -OCH₃ | -OH | -H | -OH | -OH | -H | -OCH₃ |

The anthocyanins, anthocyanidins with sugar group(s), are mostly 3-glucosides of the anthocyanidins. The anthocyanins are subdivided into the sugar-free anthocyanidin aglycones and the anthocyanin glycosides. As of 2003 more than 400 anthocyanins had been reported while more recent literature (early 2006), puts the number at more than 550 different anthocyanins. The difference in chemical structure that occurs in response to changes in pH is the reason why anthocyanins are often used as pH indicator, as they change from red in acids to blue in bases.

Anthocyanins are thought to be subject to physiochemical degradation in vivo and in vitro. It is generally known that structure, pH, temperature, light, oxygen, metal ions, intramolecular association, and intermolecular association with other compounds (co-pigments, sugars, proteins, degradation products, etc.) are known to affect the colour and stability of anthocyanins. It has been demonstrated that B-ring hydroxylation status and pH mediate the degradation of anthocyanins to their phenolic acid and aldehyde constituents. Indeed, significant portions of ingested anthocyanins are likely to degrade to phenolic acids and aldehyde in vivo, following oral consumption. This characteristic confounds scientific isolation of specific anthocyanin mechanisms in vivo.

Cancer research on anthocyanins is the most advanced, where black raspberry (*Rubus occidentalis* L.) preparations were first used to inhibit chemically induced cancer of the rat esophagus by 30-60% and of the colon by up to 80%. Effective at both the initiation and promotion/progression stages of tumour development, black raspberries are a practical research tool and a promising therapeutic source, as they contain the richest contents of anthocyanins among native North American Rubus berries. Work on laboratory cancer models has shown that black raspberry anthocyanins inhibit promotion and progression of tumour cells by
- stalling growth of pre-malignant cells;
- accelerating the rate of cell turnover, called apoptosis, effectively making the cancer cells die faster;
- reducing inflammatory mediators that initiate tumour onset;
- inhibiting growth of new blood vessels that nourish tumours - a process called angiogenesis; an
- minimising cancer-induced DNA damage.

On a molecular level, berry anthocyanins were shown to turn off genes involved with tumour proliferation, inflammation and angiogenesis, while switching on apoptosis. In 2007, black raspberry studies entered the next pivotal level of research - the human clinical trial - for which several approved studies are underway to examine anti-cancer effects of black raspberries and cranberries on tumours in the esophagus, prostate and colon. For these reasons, the preferred source for anthocyanins forming component (b) is an extract or fruit juice obtained from
- **Açai fruits** (*Euterpe oleracea*);
- **Aronia** (e.g. *Aronia arbutifolia, Aronia melanocarpa; Aronia prunifolia);*
- **Billberries** (e.g. *Vaccinium myrtillus*);
- **Black and Red currents** (e.g. *Ribes rubrum, Ribes spciatum, Ribes alpinum, Ribes schlechtendalii, Ribes multiflorum, Ribes petraeum, Ribes trite, Ribes nigrum*);
- **Blackberries** (*Rubus sp*.);
- **Black Carrots** (*Daucus carota*);
- **Black Tomatoes** (*Solanum lycopersicum*);
- **Blood oranges** (*Citrus sinensis*);
- **Blueberries** (*Vaccinium corymbosum*, *Vaccinium angustifolium*);
- **Blackthorn** (*Prunus spinosa*);
- **Bog Bilberries** (*Vaccinium ulginosum*);
- **Cloudberries** (*Rubus chamaemorus*);
- **Cranberries** (e.g. *Vaccinium oxycoccos*, *Vaccinium microcarpus*, *Vaccinium macrocarpus*, *Vaccinium erythrocarpus);*
- **Crowberries** (e.g. *Empetrum nigrum, Empetrum eamesii, Empetrum rumbrum, Empetrum hermaphroditum);*
- **Elderberries** (e.g. *Sambucus nigra*, *Sambucus racemosa*);
- **Hibiscus** *(Hibiscus sabdariffa*, *Roselle)*;
- **Lingonberries** (e.g. *Vaccinium vitus idaea*);
- **Magellan Barberries** ("Calafate", *Berberis microphylla*, *Breberis buxifolia*);
- **Maqui berries** (e.g. *Aristotelia chilensis*);
- **Mountain Bilberries** (*Vaccinium membranaceum*);
- **Prunes and prune plums** (*Prunus domestica*);
- **Raspberry** (*Rubus idaeus*, *Rubus occidentalis*);
- **Red Gooseberries** (*Ribes uva-crispa, Ribes grossularia*);
- **Red grapes** (*Vitis vinifera*, *Vitis labrusca*, *Vitis riparia*, *Vitis rotundifolia*);
- **Rowanberries** (*Sorbus aucuparia*);
- **Sour cherries** (e.g. *Prunus avium*, *Prunus cerasus*);
- **Strawberries** (*Fragaria ananassa*);
- **Sweet Cherries** (*Prunus avium*);
- **Tayberries** (*Rubus X*)
or their mixtures. Particular preferred are extracts or juices from bilberries, black raspberries or cranberries or their mixtures.

### Prebiotics

In another embodiment of the present invention the dietary supplement compositions may include prebiotics. Prebiotics are defined as non-digestible food ingredients that may beneficially affect the host be selectively stimulating the growth and/or the activity of a limited number of bacteria in the colon. Adding prebiotics to the compositions leads to another inprovement of the stability of the anthocyanins against degradation within the intestine. The following describes in particular various oligosaccharides which can be taken into account as suitable prebiotics (component c):
- ***Fructooligosaccharides**.* Fructooligosaccharides or FOS typically refer to short-chain oligosaccharides comprised of D-fructose and D-glucose, containing from three to five monosaccharide units. FOS, also called neosugar and short-chain FOS, are produced on a commercial scale from sucrose using a fungal fructosyltransferase enzyme. FOS are resistant to digestion in the upper gastrointestinal tract. They act to stimulate the growth of *Bifidobacterium* species in the large intestine. FOS are marketed in the United States in combination with probiotic bacteria and in some functional food products.
- ***Inulins**.* Inulins refer to a group of naturally-occurring fructose-containing oligosaccharides. Inulins belong to a class of carbohydrates known as fructans. They are derived from the roots of chicory *(Cichorium intybus)* and Jerusalem artichokes. Inulins are mainly comprised of fructose units and typically have a terminal glucose. The bond between fructose units in inulins is a beta-(2-1) glycosidic linkage. The average degree of polymerisation of inulins marketed as nutritional supplements is 10 to 12. Inulins stimulate the growth of *Bifidobacterium* species in the large intestine.
- ***Isomaltooligosaccharides**.* Isomaltooligosaccharides comprise a mixture of alpha-D-linked glucose oligomers, including isomaltose, panose, isomaltotetraose, isomalto-pentaose, nigerose, kojibiose, isopanose and higher branched oligosaccharides. Isomaltooligosaccharides are produced by various enzymatic processes. They act to stimulate the growth of *Bifidobacterium* species and *Lactobacillus* species in the large intestine. Isomalto oligosaccharides are marketed in Japan as dietary supplements and in functional foods. They are being developed in the United States for similar uses.
- ***Lactilol**.* Lactilol is a disaccharide analogue of lactulose. Its pharmaceutical use is in the treatment of constipation and hepatic encephalopathy. Lactilol is also used in Japan as a prebiotic. It is resistant to digestion in the upper gastrointestinal tract and it is fermented by a limited number of colonic bacteria, resulting in an increase in the biomass of bifidobacteria and lactobacilli in the colon. Lactilol is known chemically as 4-0-(beta-D-galactopyranosyl)-D-glucitol. Lactilol is not approved for the treatment of hepatic encephalopathy or constipation in the U.S., and its use as a prebiotic is considered experimental. Lactilol is used in Europe as a food sweetener.
- ***Lactosucrose**.* Lactosucrose is a trisaccharide comprised of D-galactose, D-glucose and D-fructose. Lactosucrose is produced enzymatically by the enzymatic transfer of the galactosyl residue in lactose to sucrose. Lactosucrose is resistant to digestion in the stomach and small intestine. It is selectively utilized by intestinal*Bifidobacterium* species resulting in significant induction of growth of these bacteria in the colon. Therefore, under physiological conditions, lactosucrose acts on the intestinal microflora as a growth factor for *Bifidobacterium* species. Lactosucrose is also known as 4G-beta-D-galactosyl-sucrose. It is widely used in Japan as a dietary supplement and in functional foods, including yoghurt. Lactosucrose is being developed in the United States for similar uses.
- ***Lactulose**.* Lactulose is a semi-synthetic disaccharide comprised of the sugars D-lactose and D-fructose. The sugars are joined by a beta-glycosidic linkage, making it resistant to hydrolysis by human digestive enzymes. Lactulose is, however, fermented by a limited number of colonic bacteria. This can lead to changes in the colonic ecosystem in favour of bacteria, such as lactobacilli and bifidobacteria, which may confer some health benefits. Lactulose is a prescription drug in the United States for the treatment of constipation and hepatic encephalopathy. It is marketed in Japan for use as a dietary supplement and in functional foods. Its use in the United States as a prebiotic substance is still experimental.
- ***Pyrodextrins**.* Pyrodextrins comprise a mixture of glucose-containing oligosaccharides that is derived from the hydrolysis of starch. Pyrodextrins have been found to promote the proliferation of *Bifidobacterium* species in the large intestine. They are resistant to digestion in the upper gastrointestinal tract. Pyrodextrins are being developed for the nutritional supplement market place.
- ***Soy oligosaccharides**.* Soy oligosaccharides refer to oligosaccharides found in soybeans and also in other beans and peas. The two principal soy oligosaccharides are the trisaccharide raffinose and the tetrasaccharide stachyose. Raffinose comprises one molecule each of D-galactose, D-glucose and D-fructose. Stachyose consists of two molecules of D-galactose, one molecule of D-glucose and one molecule of D-fructose. Soy oligosaccharides act to stimulate the growth of *Bifidobacterium* species in the large intestine. They are marketed in Japan as dietary supplements and in functional foods. They are being developed in the United States for similar uses.
- ***Transgalactooligosaccharides.*** Transgalactooligosaccharides (TOS) are a mixture of oligosaccharides consisting of D-glucose and D-galactose. TOS are produced from D-lactose via the action of the enzyme beta-galactosidase obtained from *Aspergillus oryzae.* TOS are resistant to digestion in the upper gastrointestinal tract and stimulate the growth of bifidobacteria in the large intestine. TOS are marketed in Japan and Europe as dietary supplements and are used in functional foods. They are being developed for similar use in the United States.
- ***Xylooligosaccharides**.* Xylooligosaccharides are comprised of oligosaccharides containing beta (1? 4) linked xylose residues. The degree of polymerisation of xylooligosaccharides is from two to four. Xylo oligosaccharides are obtained by enzymatic hydrolysis of the polysaccharide xylan. They are marketed in Japan as prebiotics and are being developed for similar use in the United States.
- ***Biopolymers**.* Suitable biopolymers like e.g. beta-glucans include those originating from plants including cereals such as oats and barley, fungi, yeast, and bacteria. In addition, microbial cell wall preparations and whole cells rich in beta glucans are also suitable sources for beta glucan preparations useful for the present invention. Monomer residues in glucans can have 1-3 and 1-4, or 1-3 and 1-6 linkages (that is the monomer units are joined through 1,3, 1,4 or 1,6 bonds) and the percent of each type can vary. Preferably, beta glucans derived from yeast, particularly from *Saccharomyces,* preferably *Saccharomyces cerevisiae,* are used for the present invention. It will be appreciated, however, that other beta glucans would also be suitable. Further examples for suitable biopolymers are chitin and its derivatives, preferably oligoglucosamin and chitosan which represent a typical hydrocolloid.

### Dietary supplements

The compositions according to the present invention are typically administered orally, however, it also possible to prepare compositions for topical applications, for example a cream, a lotion or an ointment. The compositions may comprise the probiotics and the anthocyanines in a weight ratio of about 99:1 to about 50:50 and more particularly about 95:15 to about 75:25. The highest synergistic effects, however, are observed at ratios of about 92:8 to about 80:20. In general, the compositions can be used in a concentration of up to about 90, particularly about 10 to about 75 and more particularly about 25 to about 50 % b.w. - calculated on the final food composition.

In case probiotics are incorporated into the compositions, the mixture may comprise them in amounts of about 5 to about 20, more particularly about 10 to about 15 % b.w. - all these amounts calculated on the total composition and on condition that they add to 100 % b.w.

The compositions may further comprise certain plant extracts, like extracts of *Camellia sinensis* (Green tea) or *Olea europensis* (Olive tree) which are rich in actives like polyphenols, oleuropein and hydroxtyrosol in amounts of typically 1 to 10 and particularly about 2 to 5 % b.w.

In a special embodiment of the present invention said dietary supplements are macro- or micro-encapsulated. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hall-crest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Glycospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers). The encapsulation of the compositions according to the present invention is preferred in case the active should be liberated at the same part of the intestine. Therefore, one skilled in the art can easily select the adequate encapsulation system by comparing the stability of the capsules under the pH-conditions of the respective part of the intestine.

### Industrial application

Another object of the present invention is related to the use of a composition, comprising
(a) at least one probiotic micro-organism and
(b) at least one anthocyanin, and optionally
(c) at least one prebiotic
   for food compositions. The compounds are blended and then added to the food basis, such as yoghurts, beverages and the like.

Another object of the present invention relates to a non-therapeutic method for improving the health status of the human body, in particular of the human intestine by oral or topical administration of a composition comprising
(a) at least one probiotic micro-organism and
(b) at least one anthocyanin, and optionally
(c) at least one prebiotic

Finally, the invention is also directed to a method for making a medicament for improving the health status of the human body, in particular of the human intestine, for example with respect to
- reduction of *Heliobacter pylon* infection,
- reduction of allergic symptoms,
- relief from constipation,
- relief from inflammatory bowel syndrome and inflammatories of the intestine,
- beneficial effects from mineral metabolism, particularly bone density and stability (osteoporosis prevention),
- reduction of cholesterol and triacylglycerol plasma concentrations, and
- prevention of intestine cancer.

### Examples

### Preparation Example E1

Soy milk was added to 15-75 parts by volume of cow milk to make 100 parts of the mixture. The mixture was then pasteurised at about 90 °C for 15 seconds and then cooled and separated into four samples. The cooled, pasteurised mixtures are then inoculated with 3 to 5 percent by volume of
- **C1**: a standard yoghurt culture,
- **C2**: a probiotic yoghurt culture having 1:1 ratio of *Lactobacillus bulgaricus*/*Bifidobacterium adolescentis,*
- **1**: a standard yoghurt mixture comprising a 9:1 mixture of said *Lactobacillus bulgaricus*/ *Bifidobacterium adolescentis* and an extract of bilberry comprising about bilberry b.w. anthocyanins,
- **2**: a standard yoghurt mixture comprising a 8:1:1 mixture of said *Lactobacillus bulgaricus*/ *Bifidobacterium adolescentis;* an extract of bilberry comprising about bilberry b.w. anthocyanins, and inulin.

The incubation was carried out at about 42 °C. After two hours thickening occurred. The fermentation was carried out for about 5.5 hours.

### Examples 1 and 2, Comparative Example C1 and C2

The average faecal excretion of lithocholic and deoxycholic acid (secondary bile acids which represent important metabolic products for the formation of colon cancer of the human body) related to 100 g fat intake per day has been studied over a period of two weeks. Table 1 reflects the results after consummation of the four yoghurt compositions according to Preparation Example 1. Examples 1 and 2 are according to the invention, examples C1 and C2 serve for comparison.

**Table 1**

| Faecal excretion of bile acids (mg/d) | | | | |
|---|---|---|---|---|
| **Faecal excretion** | **C1** | **C2** | **1** | **2** |
| Lithocholic acid | 305 ± 100 | 200 ± 80 | 180 ± 80 | 160 ± 80 |
| Deoxycholic acid | 580 ±+ 100 | 390 ± 100 | 350 ± 100 | 340 ± 100 |

The results indicate that adding anthocyanins to the probiotic micro-organisms leads to a significant decrease of secondary bile acids compared to the results taking the probiotics alone. Adding prebiotics to the composition even improves the results.

## Claims

1. A dietary supplement composition, comprising
(a) at least one probiotic micro-organism and
(b) at least one anthocyanin or a plant extract or a fruit juice comprising anthocyanins, and optionally
(c) at least one prebiotic
on condition that the compositions are free of milk fermentation products.

2. The composition of Claim 1, wherein said probiotic micro-organisms (component a) are selected from the group consisting of lactic acid bacteriae and bifidobacteriae.

3. The composition of Claim 1, wherein said anthocyanins are selected from the group consisting of auratinidin, cyaniding, delphinidin, europinidin, luteolinidin, pelargonidin, malvidin, peonidin, petunidin, rosinidin and their mixtures.

4. The composition of Claim 1, wherein said extracts or juices represent extracts or juices of açai fruits, aronia, black and red currents, bilberries, blackberries, black carrots, black tomatoes, blood oranges, blueberries, blackthorn, bog bilberries, cloudberries, cranberries, crowberries, elderberries, hibiscus, lingonberries, magellan barberries, maqui berries, mountain bilberries, prunes and prune plums, raspberries, red gooseberries, red grapes, rowanberries, sour cherries, strawberries, sweet cherries, tayberries or their mixtures.

5. The composition of Clam 1, wherein said prebiotics are selected from the group consisting of fructooligosaccharides, inulins, isomaltooligosaccharides, lactilol, lactosucrose, lactulose, pyrodextrins, soy oligosaccharides, transgalactooligosaccharides, xylooligosaccharides, biopolymers and their mixtures.

6. The composition of Claim 1, wherein said mixtures are macro- or micro-encapsulated.

7. A method for making a food product by adding the composition of Claim 1 to a food base.

8. A non-therapeutic method for improving the health status of the human body by oral or topical administration of the composition of Claim 1.

9. A method for making a medicament for the reduction of *Heliobacter pylon* infection, by blending at least one probiotic micro-organism, at least one anthocyanin and optionally at least one prebiotic.

10. A method for making a medicament for the reduction of allergic symptoms, by blending at least one probiotic micro-organism, at least one anthocyanin and optionally at least one prebiotic.

11. A method for making a medicament for the relief from constipation, by blending at least one probiotic micro-organism, at least one anthocyanin and optionally at least one prebiotic.

12. A method for making a medicament for the relief from inflammatory bowel syndrome and inflammatories of the intestine, by blending at least one probiotic micro-organism, at least one anthocyanin and optionally at least one prebiotic.

13. A method for making a medicament for the prevention of osteoporosis, by blending at least one probiotic micro-organism, at least one anthocyanin and optionally at least one prebiotic.

14. A method for making a medicament for the reduction of cholesterol and triacylglycerol plasma concentrations, by blending at least one probiotic micro-organism, at least one anthocyanin and optionally at least one prebiotic.

15. A method for making a medicament for the prevention of intestine cancer, by blending at least one probiotic micro-organism, at least one anthocyanin and optionally at least one prebiotic.
